# EUROPÄISCHE PATENTANMELDUNG

(11) **EP 1 964 540 A2**
(43) Veröffentlichungstag der Anmeldung: **03.09.2008**
(21) Anmeldenummer: 07024094.0
(22) Anmeldetag: 12.12.2007
(51) Int. Cl.: A61K 8/06, A61K 8/31, A61K 8/33, A61K 8/35, A61K 8/37, A61K 8/60, A61K 8/81, A61Q 19/04

(54) **Wirkstoff-stabilisierte O/W-Emulsion**

(30) Priorität: 28.02.2007 DE 102007010110; 10.12.2007 DE 102007059679
(71) Anmelder: Henkel AG & Co. KGaA, 40589 Düsseldorf (DE)
(72) Erfinder: Stadler, Iris Marina, 47249 Duisburg (DE); Bialasinski, Leszek, 40883 Ratingen (DE); Waldmann-Laue, Marianne, 40789 Monheim (DE)

(57) **Zusammenfassung**

Gegenstand der vorliegenden Anmeldung sind Öl-in-Wasser-Formulierungen (O/W-Formulierungen), die einen selbstbräunenden Wirkstoff enthalten, deren Verwendung als Hautpflegemittel, insbesondere als Haut- oder Haarbräunungsmittel, und ein Verfahren zu deren Stabilisierung.

## Beschreibung

Die vorliegende Anmeldung betrifft Öl-in-Wasser-Formulierungen (O/W-Formulierungen), die einen selbstbräunenden Wirkstoff enthalten, deren Verwendung als Hautpflegemittel, insbesondere als Haut- oder Haarbräunungsmittel, und ein Verfahren zu deren Stabilisierung.

### Stand der Technik

O/W-Formulierungen, die einen selbstbräunenden Wirkstoff enthalten, sind dem Fachmann seit langem bekannt. Ein großes Problem, mit dem der Fachmann bei der Formulierung von selbstbräunenden Kosmetika konfrontiert ist, besteht in der hohen Reaktivität der selbstbräunenden Wirkstoffe. Als selbstbräunende Wirkstoffe sind vor allem Dihydroxyaceton und Erythrulose bekannt. Strukturverwandte Polyolverbindungen sind aber ebenfalls gebräuchlich.

Die selbstbräunenden Wirkstoffe führen bei unsachgemäßer Formulierung zu einer Verfärbung des kosmetischen Produktes, was dieses erstens unansehnlich und zweitens unwirksam macht, denn der selbstbräunende Wirkstoff soll ja erst nach der Applikation auf Haut oder Haar mit den Keratinbausteinen reagieren. Diese - weitgehend undefinierten - Reaktionen im Sinne einer Maillard-Reaktion rufen nach und nach eine Bräunung der Haut oder des Haars hervor.

Aufgabe der vorliegenden Erfindung war es, O/W-Emulsionen zur Verfügung zu stellen, die langzeitstabil mit selbstbräunenden Wirkstoffen formuliert werden können.

Eine weitere Aufgabe war es, O/W-Emulsionen mit selbstbräunenden Wirkstoffen zu formulieren, die ein angenehmes, sensorisch leichtes Profil und gute Pflegeeffekte aufweisen und bei Applikation auf der Haut nicht brechen.

Ein weiterer Aspekt der Aufgabe war es, möglichst irritationsfreie O/W-Emulsionen mit selbstbräunenden Wirkstoffen bereitzustellen.

Überraschend wurde nun gefunden, dass sich diese Eigenschaften erreichen lassen, wenn man O/W-Emulsionen formuliert, die eine Kombination von ausgewählten Wachsen und Ölen enthalten.

### Beschreibung

Gegenstand der vorliegenden Anmeldung sind Öl-in-Wasser-Emulsionen, enthaltend (a) mindestens einen selbstbräunenden Wirkstoff, (b) 0,1 - 10 Gew.-% wenigstens einer Wachskomponente mit einem Schmelzpunkt von wenigstens 30°C, ausgewählt aus der Gruppe der Pentaerythritester, der Dipentaerythritester und/oder der Tripentaerythritester sowie Mischungen hiervon, (c) 1-30 Gew.-% wenigstens einer bei 25°C flüssigen Ölkomponente und (d) 40-95 Gew.-% Wasser.

Zusammensetzungen dieser Art sind langzeitstabil und hinterlassen bei der Anwendung ein glattes und weiches Gefühl mit sehr guten Pflegeeigenschaften. Sie sind leicht verteilbar, ziehen gut auf die Haut auf, hinterlassen ein geringes öliges oder fettendes, sondern vielmehr samtiges Hautgefühl.

In einer bevorzugten Ausführungsform enthalten die erfindungsgemäßen Zusammensetzungen keine zusätzlichen anionischen und kationischen Tenside/Emulgatoren. Dadurch haben die Zusammensetzungen ein geringes Irritationspotenzial.

Die erfindungsgemäßen O/W-Emulsionen weisen vorzugsweise eine Viskosität von 50000 bis 500000 mPa s auf, gemessen bei 20°C mit einem Brookfield-Viskosimeter RVF, Spindel TE, mit Helipath bei 4 Umdrehungen pro Minute.

### Pentaerythritester, Dipentaerythritester und/oder Tripentaerythritester als Wachskomponente

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30 °C ohne Zersetzung. Sie sind schon wenig oberhalb des Schmelzpunktes niedrigviskos und nicht fadenziehend und zeigen eine stark temperaturabhängige Konsistenz und Löslichkeit.

Erfindungsgemäß obligatorisch ist eine Wachskomponente oder ein Gemisch von Wachskomponenten, die bei 30 °C oder darüber schmilzt/schmelzen und ausgewählt ist/sind aus der Gruppe der Ester des Pentaerythrits, des Dipentaerythrits und des Tripentaerythrits. Die Ester des Pentaerythrits sind bevorzugt.

Die vorgenannten Ester des Pentaerythrits, Dipentaerythrits und/oder Tripentaerythrits sind in den erfindungsgemäßen Zusammensetzungen in einer Gesamtmenge von 0,1 - 10 Gew.% enthalten. In einer bevorzugten Ausführungsform der Erfindung liegt der Gehalt dieser Pentaerythrit-basierten Wachskomponente bei 0,2 - 5 Gew.-%, besonders bevorzugt bei 0,5 - 4 Gew.% und insbesondere 1 - 2 Gew.-%, jeweils bezogen auf die Gesamtzusammensetzung. In den genannten Mengenbereichen ist das sensorische Gesamtprofil der erfindungsgemäßen Öl-in-Wasser-Emulsionen optimal. Eine weitere bevorzugte Ausführungsform der erfindungsgemäßen Öl-in-Wasser-Emulsionen ist dadurch gekennzeichnet, dass die Wachskomponente oder das Gemisch aus Wachskomponenten (b) einen Schmelzpunkt zwischen 40 °C und 80 °C aufweist, vorzugsweise zwischen 40 und 60 °C, da sich in diesem Bereich die besten sensorischen Effekte ergeben.

In einer weiteren bevorzugten Ausführungsform der Erfindung ist die Wachskomponente (b) ausgewählt aus der Gruppe der Ester gesättigter oder ungesättigter und/oder verzweigter oder unverzweigter C6-C24-Fettsäuren, vorzugsweise C14-C22-Fettsäuren des Pentaerythrits, des Dipentaerythrits und/ oder Tripentaerythrits, wobei weniger als 0.3 Gew.-% C17-Fettsäureester enthalten sind. Die Ester müssen einen Schmelzpunkt von wenigstens 30°C aufweisen. Es kann sich auch um Mischester, beispielsweise langkettiger und kurzkettiger Fettsäuren, handeln, solange sie den geforderten Schmelzpunkt aufweisen. Eine weitere bevorzugte Ausführungsform der O/W-Emulsion ist dadurch gekennzeichnet, dass die Wachskomponente (b) ausgewählt ist aus der Gruppe der Ester, die durch Umsetzung des Pentaerythrits oder Dipentaerythrits mit einem Fettsäuregemisch, enthaltend 40 - 50 Gew.-% C16-Fettsäure und 45 - 55 Gew.-% C18-Fettsäure, erhältlich sind. Besonders bevorzugt sind Ester linearer, unverzweigter C16/C18-Fettsäuren.

Eine weitere bevorzugte Ausführungsform der erfindungsgemäßen Öl-in-Wasser-Emulsionen enthält wenigstens eine Wachskomponente (b), ausgewählt aus der Gruppe der Ester des Pentaerythrits mit einem Anteil an 5 - 35 Gew.-% Monoester, 20 - 50 Gew.-% Diester und 25 - 50 Gew.-% Triester, und ggf. Tetraester. Ganz besonders bevorzugt sind Ester, die durch Umsetzung des Pentaerythrits mit einem Fettsäuregemisch, enthaltend 40 - 50 Gew.-% C16-Fettsäure und 45 - 55 Gew.-% C18-Fettsäure, erhältlich sind und die folgende Esterverteilung aufweisen: 12 - 19 Gew.-% Monoester, 25 - 35 Gew.-% Diester, 30 - 40 Gew.-% Triester und 6 - 11 Gew.-% Tetraester.

Erfindungsgemäß besonders bevorzugt ist das Handelsprodukt Cutina PES (ex Cognis), INCI-Bezeichnung Pentaerythrityl Distearate

### Ölkörper

Die erfindungsgemäßen Öl-in-Wasser-Emulsionen enthalten weiterhin obligatorisch 1 - 30 Gew.-%, bezogen auf die Gesamtzusammensetzung, mindestens einer bei 25°C flüssigen Ölkomponente. Vorzugsweise ist/sind die Ölkomponente(n) in einer Gesamtmenge von 3 - 20 Gew.-%, insbesondere 5 - 15 Gew.-% und besonders bevorzugt 7 - 12 Gew.-%, bezogen auf das Gesamtgewicht der erfindungsgemäßen Öl-in-Wasser-Emulsion, enthalten.

Als Ölkörper sind beispielsweise die nachstehend genannten Verbindungsklassen geeignet, soweit diese bei 25°C flüssig sind. Hierzu zählen u.a. Guerbetalkohole auf Basis von Fettalkoholen mit 6 - 18, vorzugsweise 8 - 10 Kohlenstoffatomen, Ester von linearen oder verzweigten, gesättigten oder ungesättigten C6-C22-Fettsäuren mit linearen oder verzweigten, gesättigten oder ungesättigten C6-C22-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylisononanoat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C18-C38-Alkylhydroxycarbonsäuren mit linearen oder verzweigten, gesättigten oder ungesättigten C6-C22-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, weiterhin Triglyceride und Triglyceridmischungen, Mono-/Di-/Triglyceridmischungen, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C-Atomen, Ester der Benzoesäure mit linearen und/oder verzweigten C6-C22-Alkoholen (z. B. Cetiol AB), lineare oder verzweigte symmetrische oder unsymmetrische Dialkylether mit 6 bis 22 Kohlenstoffatomen pro Alkylgruppe, wie z. B. Di-n-octyl-ether (Cetiol OE), Ringöffnungsprodukte von epoxidierten Fettsäureestern mit Polyolen, Kohlenwasserstoffe wie Paraffin- oder Mineralöle, Siliconöle, Oligo-oder Polyalphaolefine, Dialkylcarbonate, Ester aus C8-C24-Fettsäuren und C8-C24-Fettalkoholen, sowie Mischungen dieser Substanzen.

Eine erfindungsgemäß bevorzugte Gruppe von Ölen sind die Dialkylcarbonate. Die Dialkylcarbonate können symmetrisch oder unsymmetrisch, verzweigt oder unverzweigt, gesättigt oder ungesättigt sein und lassen sich nach Umesterungsreaktionen, die aus dem Stand der Technik hinlänglich bekannt sind, herstellen. Erfindungsgemäß besonders geeignet sind Dialkylcarbonate mit Alkylketten, die 6 bis 24 Kohlenstoffatome aufweisen, insbesondere Di-n-octylcarbonat oder Di-(2-ethylhexyl)carbonat oder ein Gemisch dieser Substanzen. Unter diesen ist das Di-n-Octylcarbonat. bevorzugt.

Weitere bevorzugte Öle sind ausgewählt aus den Estern von linearen oder verzweigten, gesättigten oder ungesättigten C6-C22-Fettsäuren mit linearen oder verzweigten, gesättigten oder ungesättigten C6-C22-Fettalkoholen, insbesondere Hexyllaurat, Isostearylisononanoat, Isostearylpalmitat, Isostearylstearat, Isostearylisostearat und Isostearyloleat.

Weitere bevorzugte Öle sind ausgewählt aus den Triglyceriden, das heißt, den Glyceryltriestern, und

Triglyceridmischungen, die bei 25 °C flüssig sind. Hierzu zählen besonders bevorzugt die Glyceryltriester der Octansäure (Caprylic Triglyceride) und der Decansäure (Capric Triglyceride).

Weitere bevorzugte Öle sind ausgewählt aus pflanzlichen Ölen, insbesondere Distelöl, Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls. Ein aufgrund seiner relativ starken Polarität besonders bevorzugtes pflanzliches Öl ist Distelöl.

Weitere bevorzugte Öle sind ausgewählt aus den Estern der Benzoesäure mit linearen und/oder verzweigten C6-C22-Alkoholen, insbesondere aus Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere bevorzugte Öle sind ausgewählt aus den verzweigten gesättigten oder ungesättigten Fettalkoholen mit 6-30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24, wobei Hexyldecanol besonders bevorzugt ist.

Mit Ausnahme der Kohlenwasserstoffe, wie Paraffin- oder Mineralöle, Oligo- oder Polyalphaolefine, und der Siliconöle werden die übrigen vorstehend aufgeführten Substanzklassen eher zu den polaren Ölen gerechnet. Kohlenwasserstoffe, wie Paraffin- oder Mineralöle, Oligo- oder Polyalphaolefine, und die Siliconöle zählen dagegen eher zu den unpolaren Ölen. Überraschend wurde festgestellt, dass sich besonders lager- und temperaturstabile Öl-in-Wasser-Emulsionen formulieren lassen, wenn die Ölkomponente mindestens ein polares Öl und mindestens ein unpolares Öl enthält, wobei die polaren Öle gegenüber den unpolaren Ölen im Überschuss vorliegen. Bevorzugt beträgt dabei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1.

Erfindungsgemäß besonders bevorzugte polare Öle sind ausgewählt aus Di-n-Octylcarbonat, Hexyllaurat, Isostearylisononanoat, Isostearylpalmitat, Isostearylstearat, Isostearylisostearat, Caprylic Triglyceride, Capric Triglyceride, Distelöl, Benzoesäure-C12-C15-alkylester, Hexyldecanol, Octyldodecanol, sowie Mischungen dieser Öle. Diese Öle werden erfindungsgemäß besonders bevorzugt mit Polydimethylsiloxanen (INCI: Dimethicone) als unpolarer Ölkomponente kombiniert.

Weitere erfindungsgemäß als eher unpolare Öle einsetzbare Kohlenwasserstoffe weisen eine Kettenlänge von vorzugsweise 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatomen aufweisen, sind besonders geeignet, und unter diesen ist ein Gemisch aus Alkanen, das wenigstens 10 Gew.% verzweigte Alkane, bezogen auf die Gesamtmenge der Alkane, enthält, besonders bevorzugt. Bevorzugt handelt es sich um verzweigte, gesättigte Alkane. Besonders gut geeignet sind Gemische aus Alkanen, die mehr als 1 Gew.% 5,8-Diethyldodecan und/oder mehr als 1 Gew.-% Didecen enthalten.

### Selbstbräunende Wirkstoffe

Erfindungsgemäß bevorzugte selbstbräunende Wirkstoffe sind ausgewählt aus Dihydroxyaceton, Erythrulose und 5,6-Dihydroxyindolin sowie insbesondere bevorzugt ausgewählt aus Kombinationen von Dihydroxyaceton und Erythrulose. Die Kombination von Dihydroxyaceton und Erythrulose führt zu einem besonders natürlich wirkenden Hautton, der mit Dihydroxyaceton allein schwieriger zu erzielen ist.

Erfindungsgemäß besonders bevorzugte Öl-in-Wasser-Emulsionen sind dadurch gekennzeichnet, dass sie mindestens einen selbstbräunenden Wirkstoff in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

Erfindungsgemäß besonders bevorzugte Mischungen von Dihydroxyaceton und Erythrulose haben ein Gewichtsverhältnis von Dihydroxyaceton zu Erythrulose von 10 : 1 bis 1 : 10, bevorzugt 5 : 1 bis 1 : 5, besonders bevorzugt 3 : 1 bis 1 : 2 und außerordentlich bevorzugt 2 : 1 bis 1 : 1.

### Gelbildner

Erfindungsgemäß bevorzugte Öl-in-Wasser-Emulsionen enthalten mindestens einen Gelbildner, bevorzugt einen Hydrogelbildner, der besonders bevorzugt aus der Gruppe der Homopolymere oder Copolymere von Acrylsäure und/oder Acrylamid und/oder Acryloyldimethyltaurat und deren Salzen oder aus einem beliebigen Gemisch dieser Substanzen ausgewählt ist. Häufig sind die marktüblichen Hydrogelbildner als wasserbasierte Polymerzubereitungen konfektioniert, die gegebenenfalls ein nichtionisches Tensid/Emulgator und gegebenenfalls ein Öl enthalten. Zu den erfindungsgemäß bevorzugten Gelbildnern gehören im Handel erhältliche Substanzen oder Polymerzubereitungen, wie beispielsweise Sepigel 305, INCl: Polyacrylamid (und) C13-14 Isoparaffin (und) Laureth-7; Sepigel 501, INCl: Acrylamid Copolymer (und) Mineralöl (und) C13-14 Isoparaffin (und) Polysorbate 85, Sepigel 502, INCl: C13-14 Isoparaffin (und) Isostearyl Isostearat (und) Sodium Polyacrylate (und) Polyacrylamid (und) Polysorbate 60; Simulgel 600, INCl: Acrylamid/Sodium Acryloyldimethyltaurate Copolymer (und) Isohexadecan (und) Polysorbat 80; Simulgel 800, INCI: Sodium Polyacryloyldimethyl Taurate (und) Isohexadecan (und) Sorbitan Oleat; Simulgel EG, INCl: Sodium Acrylate/Acryloyldimethyl Taurate Copolymer (und) Isohexadecan (und) Polysorbate 80; Simulgel EG-SL (oder auch Simulgel EPG), INCl: Sodium Acrylate/Acryloyldimethyl Taurate Copolymer (und) Polyisobutene (und) Caprylyl/Capryl Glucosid; Simulgel NS, INCl: Hydroxyethyl Acrylate (und) Sodium Acryloyldimethyl Taurate Copolymer (und) Squalane (und) Polysorbate 60; Aristoflex AVC, INCl: Ammonium Acryloyldimethyltaurate/VP Copolymer; Aristoflex AVC-1, INCl: Ammonium Acryloyldimethyltaurat / Vinyl Formamid Copolymer; Aristoflex HMB, INCl: Ammonium Acryloyldimethyltaurate / Beheneth-25 Methacrylate Copolymer, Salcare SC 91, INCl: Sodium Acrylate Copolymer (und) Mineralöl (und) PPG-1 Trideceth-6; Salcare AST, INCl: Sodium Acrylate Copolymer (und) Glycine Soja (und) PPG-1 Trideceth-6; Pemulen TR-1, INCl: Acrylates / C10-30 Alkyl Acrylate Crosspolymer; Pemulen TR-2: Acrylates / C10-30 Alkyl Acrylate Crosspolymer; Carbopol 980, INCl: Carbomer (z.B. Homopolymere von Acrylsäure, vernetzt mit einem Allylether von Pentaerythritol, einem Allylether von Sucrose oder einem Allylether von Propylen); Carbopol ETD 2020, INCl: Acrylates / C10-30 Alkyl Acrylate Crosspolymer; Carbopol Ultrez 10, INCl: Carbomer; Rheocare ATH, INCl: Sodium Polyacrylate (und) Ethylhexyl Stearate (und) Trideceth-6; Rheocare ATC, INCI: Acrylamid / Sodium Acrylate Copolymer (und) Mineralöl (und) Trideceth-6.

Die Polymere können vernetzt oder unvernetzt sein. Vorzugsweise werden vernetzte Polymere eingesetzt.

Erfindungsgemäß bevorzugt sind Polyacrylate und Polyacryloyldimethyltaurate. Besonders bevorzugt sind Polyacrylate, insbesondere deren Natriumsalze, sowie Polyacryloyldimethyltaurate und deren Natrium- und/oder Ammoniumsalze. Die Natriumsalzform ist dabei besonders bevorzugt. Ein erfindungsgemäß besonders bevorzugtes Polymer ist unter dem Namen Cosmedia SP und Cosmedia SPL (Cognis) im Handel. Weiterhin besonders bevorzugt sind die Polyacryloyldimethyltaurat-Zubereitungen Sepigel 305, Simulgel NS, Simulgel EG und Simulgel EPG (siehe vorstehende Erläuterung). Die Polymere werden erfindungsgemäß bevorzugt in einer Gesamtmenge von 0,05 - 1 Gew.-%, besonders bevorzugt 0,1 - 0,8 Gew.%, insbesondere von 0,2 - 0,6 Gew.-% und ganz besonders bevorzugt von 0,3 - 0,4 Gew.-%, jeweils bezogen auf die Polymeraktivsubstanz in der Gesamtzusammensetzung, eingesetzt.

Eine bevorzugte Ausführungsform der erfindungsgemäßen Öl-in-Wasser-Emulsion enthält (a) mindestens einen selbstbräunenden Wirkstoff, (b) 0,1 - 10 Gew.-% wenigstens eines C16-C18-Esters des Pentaerythrits und/oder des Dipentaerythrits, (c) 1 - 30 Gew.-% wenigstens einer bei 25 °C flüssigen Ölkomponente, (d) 0,05 - 5 Gew.-% wenigstens eines Polyacrylats und (e) 60 - 95 Gew.-% Wasser.

Eine weitere bevorzugte Ausführungsform der erfindungsgemäßen O/W-Emulsion enthält (a) mindestens einen selbstbräunenden Wirkstoff, (b) 0,1 - 10 Gew.-% wenigstens eines C16-C18-Esters des Pentaerythrits und/oder des Dipentaerythrits, (c) 1 - 30 Gew.-% wenigstens einer bei 25 °C flüssigen Ölkomponente, (d) 0,05 - 5 Gew.-% wenigstens eines Polyacrylats und (e) 40 - 95 Gew.-% Wasser.

Das Polyacrylat (d) ist vorzugsweise ein Natriumpolyacrylat.

Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäßen Öl-in-Wasser-Emulsion enthält (a) mindestens einen selbstbräunenden Wirkstoff, (b) 0,1 - 10 Gew.-% wenigstens eines C16-C18-Esters des Pentaerythrits und/oder des Dipentaerythrits, (c) 1-30 Gew.% wenigstens einer bei 25 °C flüssigen Ölkomponente, (d) 0,05 - 5 Gew-% wenigstens eines Acryloyldimethyltaurats und (e) 40 - 95 Gew.-% Wasser.

Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäßen Öl-in-Wasser-Emulsion enthält (a) ) mindestens einen selbstbräunenden Wirkstoff, (b) 0,5 - 2 Gew.-% wenigstens eines Esters, den man durch Umsetzung des Pentaerythrits und/oder des Dipentaerythrits mit einem Fettsäuregemisch enthaltend 40 - 50 Gew.-% C16-Fettsäure und 45-55 Gew.-% C18-Fettsäure, erhält, (c) 7-12 Gew.-% wenigstens einer Ölkomponente, ausgewählt aus bei 25 °C flüssigen Fettsäureestern, Triglyceriden, Dialkylcarbonaten, Kohlenwasserstoffen, Dialkylethern oder einem beliebigen Gemisch dieser Substanzen, (d) 0,5 - 2 Gew-% wenigstens eines Natriumpolyacrylats und (e) 60 - 95 Gew.-% Wasser.

Vorzugsweise sind die unter (b) genannten Ester Pentaerythritester mit folgender Esterverteilung: 12 -19 Gew.-% Monoester, 25 - 35 Gew.-% Diester, 30 - 40 Gew.-% Triester und 6 - 11 Gew-% Tetraester.

Weiterhin wurde überraschend festgestellt, dass die erfindungsgemäßen Öl-in-Wasser-Emulsionen auch die problemlose Einarbeitung von organischen und/oder anorganischen UV-Filtern ermöglichen, unter gleichzeitiger Lager- und Temperaturstabilität, und dabei ein sehr gutes sensorisches Hautgefühl aufweisen. Selbstbräunungszusammensetzungen werden von vielen Verbrauchern als Alternative zum Sonnenbad eingesetzt, um die Exposition mit den schädigenden UV-Strahlen zu vermeiden. Hier ist es besonders sinnvoll, den Wunsch des Verbrauchers nach größtmöglichem Lichtschutz nachzukommen und die Wirkung des Selbstbräunens mit einem Lichtschutzfaktor zu kombinieren.

Erfindungsgemäß besonders bevorzugte Öl-in-Wasser-Emulsionen sind deshalb dadurch gekennzeichnet, dass sie mindestens eine anorganische und/oder mindestens eine organische UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoe-säureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methyl-benzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethyl-siloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethyl-siloxy-silylpropyloxy)-2-hydroxy]-phenyl}-6-(4-methoxyphenyl)-1,3,5-triazin, 2,4-Bis-{[4-(2-methylpro-penyloxy)-2-hydroxy]-phenyl}-6-(4-methoxyphenyl)-1,3,5-triazin, 2,4-Bis-{[4-(1',1',1',3',5',5',5'-Hepta-methylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI-Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCl-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenyl-zimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß ist mindestens eine organische UV-Filtersubstanz bevorzugt in einer Gesamtmenge von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.

Erfindungsgemäß ist mindestens eine anorganische UV-Filtersubstanz bevorzugt in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weiterhin wurde überraschend festgestellt, dass die erfindungsgemäßen Öl-in-Wasser-Emulsionen bevorzugt durch nichtionische Emulgatoren oder Tenside weiter lager- und temperaturstabil gemacht werden können und dabei ein sehr gutes sensorisches Hautgefühl aufweisen.

Erfindungsgemäß besonders bevorzugte Öl-in-Wasser-Emulsionen sind deshalb dadurch gekennzeichnet, dass sie mindestens einen nichtionischen Öl-in-Wasser-Emulgator enthalten.

Typische Beispiele für nichtionische Öl-in-Wasser-Emulgatoren sind Fettalkoholpolyglycolether, Polyglycerinester, Fettsäurepolyglycolester, Fettsäureamid-polyglycolether, Fettaminpolyglycolether, alkoxylierte Triglyceride, Mischether, Alk(en)yloligoglykoside bzw. Glucoronsäurederivate - gegebenenfalls partiell oxidiert -, Fettsäure-N-alkylglucamide, Polyolfettsäureester, Zuckerester, Sorbitanester, Polysorbate und Aminoxide. Sofern die nichtionischen Öl-in-Wasser-Emulgatoren Polyglycoletherketten enthalten, können diese eine konventionelle, vorzugsweise jedoch eine eingeengte Homologenverteilung aufweisen.

Bevorzugte nichtionische Öl-in-Wasser-Emulgatoren sind ausgewählt aus ethoxylierten C₈-C₂₄-Alkanolen mit durchschnittlich 5-100 Mol Ethylenoxid pro Mol, ethoxylierten C₈-C₂₄-Carbonsäuren mit durchschnittlich 5-100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Feftsäureresten verestert, sofern sie einen HLB-Wert von mehr als 7 aufweisen, sowie Mischungen der vorgenannten Substanzen, wobei der gewichtsmittlere HLB-Wert des Öl-in-Wasser-Emulgatorsystems bevorzugt 11 - 17, besonders bevorzugt 12 - 15 und außerordentlich bevorzugt 13 - 14 beträgt.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 5 - 100 Mol, vorzugsweise 10 - 30 Mol, Ethylenoxid an technische Fettalkohole mit 12 - 18 Kohlenstoffatomen, wie beispielsweise Kokos-, Palm-, Palmkern- oder Talgfettalkohol, sind geeignet.

Besonders bevorzugte nichtionische Öl-in-Wasser-Emulgatoren sind ausgewählt aus der Gruppe, bestehend aus Ceteth-12, Ceteth-20, Ceteth-30, Steareth-12, Steareth-20, Steareth-30, Laureth-12 und Beheneth-20, sowie Mischungen hiervon.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 5 - 100 Mol, vorzugsweise 10 - 30 Mol, Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen.

Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1-2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Die nichtionischen Öl-in-Wasser-Emulgatoren können u.a. auch durch handelsübliche gelbildende Polymerzubereitungen eingetragen werden, wenn auch nur in geringen Mengen, bezogen auf die erfindungsgemäße Öl-in-Wasser-Emulsion.

Besonders bevorzugte erfindungsgemäße Öl-in-Wasser-Emulsionen sind dadurch gekennzeichnet, dass mindestens ein nichtionischer Öl-in-Wasser-Emulgator in einer Gesamtmenge von 0,2 - 10 Gew.-%, bevorzugt 0,6 - 5 Gew.-%, besonders bevorzugt 1 - 4 Gew.-%, außerordentlich bevorzugt 2 - 3 Gew.%, jeweils bezogen auf die Gesamtzusammensetzung, enthalten ist.

Die Anwesenheit von Proteinen und Proteinhydrolysaten ist erfindungsgemäß weniger bevorzugt, da diese Verbindungen mit den Selbstbräuner-Wirkstoffen reagieren und zu einer Verfärbung und

Inaktivierung des Produktes führen können. Beide Substanzklassen können allerdings problemlos nebeneinander enthalten sein, falls mindestens einer der beiden Wirkstoffe in verkapselter Form vorliegt. Dies führt allerdings zu einer Verteuerung der Rohstoffkosten.

Ein weiterer Gegenstand der vorliegenden Anmeldung ist die Verwendung der erfindungsgemäßen Öl-in-Wasser-Emutsionen zur Körperpflege und/oder zur Bräunung der Haut.

Ein weiterer Gegenstand der Erfindung ist die Verwendung von Estern des Pentaerythrits, des Dipentaeryttrits und/oder Tripentaerythrits zur Stabilisierung von Öl-in-Wasser-Emulsionen, die mindestens einen selbstbräunenden Wirkstoff enthalten.

### Weitere fakultative Hilfs- und Zusatzstoffe

Je nach Applikationszweck enthalten die kosmetischen Formulierungen eine Reihe weiterer Hilfs- und Zusatzstoffe wie beispielsweise ionische Emulgatoren/Tenside, weitere Wachs- oder Lipidkomponenten, weitere Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenre-pellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als weitere Wachs- oder Lipidkomponenten können Fette und fettähnliche Substanzen mit wachsartiger Konsistenz eingesetzt werden. Die erfindungsgemäß als Fette und fettähnliche Substanzen mit wachsartiger Konsistenz bezeichneten Komponenten weisen inen Schmelzpunkt (unter Normalbedingungen = 1013 mbar) von mindestens 30 °C auf. Hierzu gehören u.a. Triglyceridfette, Mono- und Diglyceride von gesättigten, linearen C12-C40-Fettsäuren, natürliche und synthetische Wachse, Fett- und Wachsalkohole, C10-C40-Fettsäuren, Ester von Fettalkoholen und Fettsäuren, die nicht bei 25°C flüssig sind, sowie Fettsäureamide und beliebige Gemische dieser Substanzen.

Unter Fetten versteht man Triacylglycerine, also die Dreifachester von Fettsäuren mit Glycerin.

Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln.

Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische.

Als zusätzliche Wachskomponenten sind insbesondere Mono- und Diglyceride bzw. Mischungen dieser Partialglyceride einsetzbar. Zu den erfindungsgemäß einsetzbaren Glyceridgemischen zählen die von Cognis vermarkteten Produkte Novata AB und Novata B (Gemisch aus C12-C18-Mono-, Di- und Triglyceriden) sowie Cutina MD oder Cutina GMS (Glycerylstearat).

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung weiter verbessern.

Erfindungsgemäß besonders bevorzugte Öl-in-Wasser-Emulsionen enthalten daher 2-10 Gew.-%, bevorzugt 4-8 Gew.%, besonders bevorzugt 5-6 Gew.% mindestens einer Lipidkomponente, ausgewählt aus Glycerylmonostearat, Glyceryldistearat und Mischungen hiervon.

Zu den erfindungsgemäß einsetzbaren Fettalkoholen zählen die linearen C12-C50-Fettalkohole, insbesondere die C12-C24-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte, unverzweigte Fettalkohole. Erfindungsgemäß einsetzbar sind auch Fettalkoholschnitte, wie sie bei der Reduktion natürlich vorkommender Fette und Öle wie z. B. Rindertalg, Erdnussöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß bevorzugt geeignet sind C14-C22-Fettalkohole, die beispielsweise von Cognis unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden.

Fettalkohole verleihen den Zusammensetzungen ein trockeneres Hautgefühl als Triglyceride und sind daher gegenüber letzteren bevorzugt.

Erfindungsgemäß besonders bevorzugte Öl-in-Wasser-Emulsionen enthalten daher 1,5-6 Gew.-%, bevorzugt 2-5 Gew.-%, besonders bevorzugt 3-4 Gew.-% mindestens einer Lipidkomponente, ausgewählt aus Myristylalkohol, Cetylalkohol, Stearylalkohol, Arachidylalkohol, Behenylalkohol und Mischungen hiervon.

Als zusätzliche Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-,Lignocerin-, Cerotin-, Melissin-, Eruca-und Elaeostearinsäure sowie substituierte Fettsäuren, wie z. B. 1 2-Hydroxystearinsäure, und die Amide oder Monoethanolamide der Fettsäuren, wobei diese Aufzählung beispielhaften und keinen beschränkenden Charakter hat.

Erfindungsgemäß verwendbar sind außerdem natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die zusätzliche Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, aus der Gruppe der mit Carbonsäuren veresterten Polyole (andere als Pentaerythrit, Dipentaerythrit oder Tripentaerythrit), insbesondere 1,2-Propylenglycol, Ethylenglycol und Glycerin, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Cetylpalmitat, Stearylstearat, Palmitylstearat, Stearylbehenat, Cetearylbehenat und Behenylbehenat einsetzbar. Aus dieser Gruppe der sogenannten Wachsester ist Cetylpalmitat besonders bevorzugt, insbesondere in Mengen von 0,2 - 1 Gew.-%, bevorzugt 0,4 - 0,8 Gew.-%, besonders bevorzugt 0,5 - 0,6 Gew.-%, jeweils bezogen auf das Gewicht der erfindungsgemäßen Öl-in-Wasser-Emulsion.

Als oberflächenaktive Stoffe können anionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 10 Gew.-% nicht. Eine bevorzugte Ausführungsform der Erfindung enthält keine anionischen oder kationischen Tenside, weist aber einen Gehalt an nichtionischen Tensiden auf.

Die nichtionischen Tenside können u.a. auch in den handelsüblichen Gelbildnern enthalten sein.

Typische Beispiele für anionische Tenside sind Seifen, Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Fettalkoholethersulfate, Glycerinethersulfate, Fettsäureethersulfate, Hydroxymischethersul-fate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Alkyl(ether)phosphate. Sofern die anionischen Tenside Polyglycoletherketten enthalten, können diese eine konventionelle, vorzugsweise- jedoch eine eingeengte Homologenverteilung aufweisen.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, alpha-Olefinsulfonate, Ethercarbonsäuren, Alkyloligoglucoside, Fettsäureglucamide, Alkylamidobetaine und/oder Amphoacetate.

Als weitere Verdickungsmittel eignen sich beispielsweise Aerosil-Typen (hydrophile Kieselsäuren), Polysaccharide, insbesondere Xantban-Gum, Guar-Guar, Agar-Agar, Alginate, Carboxymethylcellulose, Hydroxyethylcellulose und Hydroxypropylcellulose, Polyvinylalkohol, Polyvinylpyrrolidon und Bentonite, wie z. B. Bentone Gel VS-5PC (Rheox).

Weitere optionale Wirkstoffe sind beispielsweise Tocopherol, Tocopherolacetat, Tocopherolpalmitat, Ascorbinsäure, beta-Glucane, Retinol, Bisabolol, Allantoin, Phytantriol, Panthenol, alpha-Hydroxycarbonsäuren (AHA-Säuren), Ceramide, Pseudoceramide, essentielle Öle, Pflanzenextrakte und Vitaminkomplexe.

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Bevorzugt sind diese Polyole ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit Xylitol, sowie Mischungen der vorgenannten Substanzen. Als Polyole geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Als Konservierungsmittel eignen sich beispielsweise Phenoxyethanol, Parabene, Pentandiol oder Sorbinsäure und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stängeln und Blättern, Früchten, Fruchtschalen, Wurzeln, Hölzern, Krätern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Farbstoffe können die für kosmetische Zwecke geeigneten und zugelassenen Substanzen verwendet werden. Beispiele sind Cochenillerot A (C.I. 16255), Patentblau V (C.1.42051), Indigotin (C.1.73015), Chlorophyllin (C.1.75810), Chinolingelb (C.1.47005), Titandioxid (C.1. 77891), Indanthrenblau RS (C.1. 69800) und Krapplack (C.1.58000). Diese Farbstoffe werden üblicherweise in Konzentrationen von 0,001 bis 0,1 Gew.-%, bezogen auf die gesamte Mischung, eingesetzt.

Die folgenden Beispiele sollen den Gegenstand der vorliegenden Anmeldung erläutern, ohne ihn hierauf zu beschränken.

### 1. Tagescreme mit Bräunungseffekt

| | |
|---|---|
| Cutina PES | 5,000000 |
| Cetiol SN | 4,000000 |
| Myritol 318 | 4,000000 |
| Distelöl | 2,000000 |
| Silikonöl 350 cSt | 1,500000 |
| Emulgade SE-PF | 6,000000 |
| Behenylalkohol | 2,000000 |
| Vitamin E Acetat | 0,500000 |
| Controx KS | 0,050000 |
| Propylparaben | 0,200000 |
| Uvinul A Plus | 2,000000 |
| Hexandiol-1,6 | 6,000000 |
| Glycerin 86% | 5,000000 |
| 1,3-Butylenglycol | 6,000000 |
| Propandiol-1,2 | 7,000000 |
| Methylparaben | 0,200000 |
| Citronensäure Monohydrat | 0,150000 |
| Trinatriumcitrat Dihydrat | 0,240000 |
| Erythrulose | 2,000000 |
| Dihydroxyaceton | 3,000000 |
| Lipochroman-6 | 0,010000 |
| D-Panthenol 75 % | 2,000000 |
| SymGlucan | 1,200000 |
| Simulgel NS | 1,250000 |
| Wasser, vollentsalzt | ad 100,0000 |

### 2. Körperlotion mit Bräunungseffekt

| | |
|---|---|
| Cutina PES | 1,000000 |
| Cetiol CC | 3,000000 |
| Benzoesäure C12-15 Alkylester | 6,000000 |
| Silikonöl 350 cSt | 0, 500000 |
| Emulgade SE-PF | 8,000000 |
| Behenylalkohol | 3,000000 |
| Vitamin E Acetat | 0,500000 |
| Controx KS | 0,050000 |
| Propylparaben | 0,200000 |
| Simulgel NS | 1,300000 |
| Hexandiol-1,6 | 6,000000 |
| Glycerin 86% pflanzlich | 5,000000 |
| Sorbit 70% | 2,000000 |
| Methylparaben | 0,200000 |
| Citronensäure Monohydrat | 0,150000 |
| Trinatriumcitrat Dihydrat Pulver fein | 0,200000 |
| DSH-C N | 5,000000 |
| Erythrulose | 1,000000 |
| Dihydroxyaceton | 2,000000 |
| D-Panthenol 75 % | 0,700000 |
| Ederline L | 2,000000 |
| SymGlucan | 1,000000 |
| Parfum | 0,250000 |
| Aluminiumstärkeoctenylsuccinat | 3,000000 |
| Wasser, vollentsalzt | ad 100,0000 |

### 3 Tagescreme mit Bräunungseffekt und Anti-Aging-Wirkstoffen

| | |
|---|---|
| Pentaerythrityl Distearate | 1,0 |
| Glyceryl Stearate | 5,0 |
| Ceteareth-20 | 1,2 |
| Ceteareth-12 | 0,6 |
| Cetearyl Alcohol | 0,6 |
| Cetyl Palmitate | 0,6 |
| Behenyl Alcohol | 3,0 |
| C12-15 Alkyl Benzoate | 6,0 |
| Dicaprylyl Carbonate | 3,0 |
| Hexyldecanol | 2,5 |
| Dimethicone | 1,5 |
| Aluminum Starch Octenylsuccinate | 3,0 |
| Dihydroxyaceton | 2,0 |
| Erythrulose | 1,0 |
| Sorbitol | 2,0 |
| 1,6-Hexandiol | 6,0 |
| Glycerin | 4,3 |
| Hydroxyethyl Acrylate/Sodium Acryloyldimethyl Taurate Copolymer | 0,4 |
| Squalane | 0,3 |
| Polysorbate 60 | 0,05 |
| Panthenol | 0,5 |
| Tocopheryl Acetate | 0,5 |
| Dimethylsilanol Hyaluronate | 0,03 |
| Tocopherol | 0,03 |
| Hydrogenated Palm Glycerides Citrate | 0,02 |
| Beta-Glucan | 0,01 |
| Parfum | 0,2 |
| Propylparaben | 0,2 |
| Methylparaben | 0,2 |
| Sodium Citrate | 0,2 |
| Citric Acid | 0,15 |
| Wasser, vollentsalzt | ad 100,0000 |

Die angegebenen Mengen beziehen sich auf Gew.-% der handelsüblichen Substanz bzw. des angegebenen Inhaltsstoffes in der Gesamtzusammensetzung.

### Zur Herstellung der Beispiele 1 - 3 wurden folgende Rohstoffe eingesetzt:

| | | |
|---|---|---|
| Handelsname | INCI-Bezeichnung | Hersteller/Lieferant |
| Cetiol CC | Dicaprylyl Carbonate | Cognis |
| Cetiol SN | Cetearyl Isononanoate | Cognis |
| Controx KS | Tocopherol, Hydrogenated Palm Glycerides Citrate | Cognis |
| Cutina PES | Pentaerythrityl Distearate | Cognis |
| DSH-C N | AQUA (WATER), Dimethylsilanol Hyaluronate | Exsymol |
| Ederline L | HEXYLDECANOL, PYRUS MALUS (APPLE) FRUIT EXTRACT | Vincience |
| Emulgade SE-PF | Glyceryl Stearate, Ceteareth-20, Ceteareth-12, Cetearyl Alcohol, Cetyl Palmitate | Cognis |
| Lipochroman-6 | DIMETHYLMETHOXY CHROMANOL | Lipotec SA |
| Myritol 318 | Caprylic/Capric Triglyceride | Cognis |
| Simulgel NS | AQUA (WATER), Hydroxyethyl Acrylate/ Sodium Acryloyldimethyl Taurate Copolymer, Squalane, Polysorbate 60 | Seppic |
| SymGlucan | AQUA (WATER), GLYCERIN, BETAGLUCAN (1 Gew.-%) | Symrise |
| Uvinul A Plus | DIETHYLAMINO HYDROXYBENZOYL HEXYLBENZOATE | BASF |

## Patentansprüche

1. Öl-in-Wasser-Emulsion, enthaltend
(a) mindestens einen selbstbräunenden Wirkstoff,
(b) 0,1 - 10 Gew.-% wenigstens einer Wachskomponente mit einem Schmelzpunkt von wenigstens 30°C, ausgewählt aus der Gruppe der Pentaerythritester, der Dipentaerythritester und/oder der Tripentaerythritester sowie Mischungen hiervon,
(c) 1 - 30 Gew.-% wenigstens einer bei 25°C flüssigen Ölkomponente und
(d) 40 - 95 Gew.-% Wasser.

2. Öl-in-Wasser-Emulsion gemäß Anspruch 1, **dadurch gekennzeichnet, dass** die Wachskomponente (b) in einer Menge von 0,2 - 5 Gew.-% der Gesamtzusammensetzung enthalten ist.

3. Öl-in-Wasser-Emulsion gemäß Anspruch 1 oder 2, **dadurch gekennzeichnet, dass** die Wachskomponente (b) ausgewählt ist aus der Gruppe der Ester gesättigter oder ungesättigter und/ oder verzweigter oder unverzweigter C6-C24-Fettsäuren, vorzugsweise C14-C22-Fettsäuren des Pentaerythrits, des Dipentaerythrits und/oder des Tripentaerythrits oder einem beliebigen Gemisch dieser Ester, wobei weniger als 0,3 Gew.-% C17-Fettsäureester enthalten sind.

4. Öl-in-Wasser-Emulsion gemäß einem der Ansprüche 1 bis 3, **dadurch gekennzeichnet, dass** die Wachskomponente (b) ausgewählt ist aus der Gruppe der Ester, die durch Umsetzung des Pentaerythrits oder des Dipentaerythrits mit einem Fettsäuregemisch, enthaltend 40 - 50 Gew.-% C16-Fettsäure und 45 - 55 Gew.-% C18-Fettsäure, erhältlich ist.

5. Öl-in-Wasser-Emulsion gemäß einem der Ansprüche 1 bis 4, **dadurch gekennzeichnet, dass** die Wachskomponente (b) ausgewählt ist aus der Gruppe der Ester des Pentaerythrits mit einem Anteil an (i) 5 - 35 Gew.-% Monoester, (ii) 20 - 50 Gew.-% Diester und (iii) 25 - 50 Gew.-% Triester, und gegebenenfalls Tetraester.

6. Öl-in-Wasser-Emulsion gemäß einem der Ansprüche 1 bis 5, **dadurch gekennzeichnet, dass** die Wachskomponente (b) ausgewählt ist aus der Gruppe der Ester des Pentaerythrits mit einem Anteil an (i) 12 - 19 Gew.-% Monoester, (ii) 25 - 35 Gew.-% Diester, (iii) 30 - 40 Gew.-% Triester und (iv) 6 - 11 Gew.-% Tetraester.

7. Öl-in-Wasser-Emulsion gemäß einem der Ansprüche 1 bis 6, **dadurch gekennzeichnet, dass** sie 0,05 - 5 Gew.-% wenigstens eines polymeren Gelbildners, ausgewählt aus der Gruppe der Homopolymere oder Copolymere von Acrylsäure und/oder Acrylamid und/oder Acryloyldimethyltaurat und deren Salzen, enthält.

8. Öl-in-Wasser-Emulsion gemäß einem der Ansprüche 1 bis 7, **dadurch gekennzeichnet, dass** sie
(a) mindestens einen selbstbräunenden Wirkstoff,
(b) 0,1 - 10 Gew.-% wenigstens eines C16-C18-Esters des Pentaerythrits und/oder des Dipentaerythrits,
(c) 1-30 Gew.-% wenigstens einer bei 25 °C flüssigen Ölkomponente
(d) 0,05 - 5 Gew-% wenigstens eines Polyacrylats und
(e) 40 - 95 Gew.-% Wasser
enthält.

9. Öl-in-Wasser-Emulsion gemäß Anspruch 8, **dadurch gekennzeichnet, dass** das Polyacrylat (d) ein Natriumpolyacrylat ist.

10. Öl-in-Wasser-Emulsion gemäß einem der Ansprüche 1 bis 9, **dadurch gekennzeichnet, dass** sie
(a) mindestens einen selbstbräunenden Wirkstoff,
(b) 0,5 - 2 Gew.-% wenigstens eines Esters, den man durch Umsetzung des Pentaerythrits und/oder des Dipentaerythrits mit einem Fettsäuregemisch, enthaltend 40 - 50 Gew.-% C16-Fettsäure und 45-55 Gew.-% C18-Fettsäure, erhält,
(c) 7-12 Gew.-% wenigstens einer Ölkomponente, ausgewählt aus bei 25 °C flüssigen Fettsäureestern, Triglyceriden, Dialkylcarbonaten, Kohlenwasserstoffen, Dialkylethern oder einem beliebigen Gemisch dieser Substanzen,
(d) 0,5 - 2 Gew-% wenigstens eines Natriumpolyacrylats und
(e) 60 - 95 Gew.-% Wasser
enthält.

11. Öl-in-Wasser-Emulsion gemäß einem der Ansprüche 1 bis 10, **dadurch gekennzeichnet, dass** sie
(a) mindestens einen selbstbräunenden Wirkstoff,
(b) 0,1 - 10 Gew.-% wenigstens eines C16-C18-Esters des Pentaerythrits und/oder des Dipentaerythrits,
(c) 1 - 30 Gew.-% wenigstens einer bei 25 °C flüssigen Ölkomponente,
(d) 0,05 - 5 Gew-% wenigstens eines Acryloyldimethyltaurats und
(e) 40 - 95 Gew.-% Wasser
enthält.

12. Öl-in-Wasser-Emulsion gemäß einem der Ansprüche 1 bis 11, **dadurch gekennzeichnet, dass** die Ölkomponente ausgewählt ist aus Fettsäureestern, Dialkylcarbonaten, Dialkylethern, Siliconölen, natürlichen Ölen und beliebigen Mischungen dieser Ölkomponenten.

13. Öl-in-Wasser-Emulsion gemäß einem der Ansprüche 1 bis 12, **dadurch gekennzeichnet, dass** die Ölkomponente mindestens ein polares Öl und mindestens ein unpolares Öl enthält, wobei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1-beträgt.

14. Öl-in-Wasser-Emulsion gemäß einem der Ansprüche 1 bis 13, **dadurch gekennzeichnet, dass** die polaren Öle ausgewählt sind aus Di-n-Octylcarbonat, Hexyllaurat, Isostearylisononanoat, Isostearylpalmitat, Isostearylstearat, Isostearylisostearat, Caprylic Triglyceride, Capric Triglyceride, Distelöl, Benzoesäure-C12-C15-alkylester, Hexyldecanol, Octyldodecanol, sowie Mischungen dieser Öle, und die unpolare Ölkomponente ausgewählt ist aus Polydimethylsiloxanen (INCI: Dimethicone).

15. ÖI-in-Wasser-Emulsion gemäß einem der Ansprüche 1 bis 14, **dadurch gekennzeichnet, dass** der mindestens eine selbstbräunende Wirkstoff ausgewählt ist aus Dihydroxyaceton, Erythrulose und Mischungen dieser Substanzen, insbesondere Mischungen mit einem Gewichtsverhältnis von Dihydroxyaceton zu Erythrulose von 10 : 1 bis 1 : 10, bevorzugt 5 : 1 bis 1 : 5, besonders bevorzugt 3 : 1 bis 1 : 2 und außerordentlich bevorzugt 2 : 1 bis 1 : 1.

16. Öl-in-Wasser-Emulsion gemäß einem der Ansprüche 1 bis 15, **dadurch gekennzeichnet, dass** mindestens eine anorganische und/oder mindestens eine organische UV-Filtersubstanz enthalten ist.

17. Öl-in-Wasser-Emulsionen gemäß einem der Ansprüche 1 bis 16, **dadurch gekennzeichnet, dass** sie mindestens einen nichtionischen Öl-in-Wasser-Emulgator enthalten, der bevorzugt ausgewählt ist aus Ceteth-12, Ceteth-20, Ceteth-30, Steareth-12, Steareth-20, Steareth-30, Laureth-12 und Beheneth-20, sowie Mischungen hiervon.

18. Öl-in-Wasser-Emulsion gemäß einem der Ansprüche 1 bis 17, **dadurch gekennzeichnet, dass** 2 - 10 Gew.-%, bevorzugt 4 - 8 Gew.-%, besonders bevorzugt 5 - 6 Gew.-% mindestens einer Lipidkomponente, ausgewählt aus Glycerylmonostearat, Glyceryldistearat und Mischungen hiervon, enthalten sind.

19. Öl-in-Wasser-Emulsion gemäß einem der Ansprüche 1 bis 18, **dadurch gekennzeichnet, dass** 1,5 - 6 Gew.-%, bevorzugt 2 - 5 Gew.-%, besonders bevorzugt 3 - 4 Gew.-% mindestens einer Lipidkomponente, ausgewählt aus Myristylalkohol, Cetylalkohol, Stearylalkohol, Arachidylalkohol, Behenylalkohol und Mischungen hiervon, enthalten sind.

20. Öl-in-Wasser-Emulsion gemäß einem der Ansprüche 1 bis 19, **dadurch gekennzeichnet, dass** keine anionischen und keine kationischen Tenside/Emulgatoren enthalten sind.

21. Verwendung einer Öl-in-Wasser-Emulsion gemäß einem der Ansprüche 1 bis 20 zur Körperpflege und/oder zur Bräunung der Haut.

22. Verwendung von Estern des Pentaerythrits, des Dipentaerythrits und/oder Tripentaerythrits zur Verbesserung der Stabilität von Öl-in-Wasser-Emulsionen, enthaltend mindestens einen selbstbräunenden Wirkstoff.
